# EUROPEAN PATENT SPECIFICATION

(11) **EP 3 331 357 B1**
(45) Date of publication and mention of the grant of the patent: **10.03.2021**
(21) Application number: 16745763.9
(22) Date of filing: 03.08.2016
(51) Int. Cl.: A01N 25/00, A01N 25/08, A01N 65/00, C12N 9/24, C12N 11/14

(54) **PREPARATION ABLE TO PRODUCE BIOPESTICIDE AND/OR REPELLENT FOR CONTROLLING PLANT PATHOGENS**
ZUSAMMENSETZUNG BEFÄHIGT ZUR HERSTELLUNG EINES BIOPESTICIDES UND/ODER REPELLENTS ZUR KONTROLLE VON PFLANZENPATHOGENEN
PREPARATION CAPABLE DE PRODUIRE UN BIOPESTICIDE ET/OU REPELLENT POUR LE CONTRÔLE DES PATHOGÈNES DES PLANTES

(30) Priority: 07.08.2015 LU 92793
(43) Date of publication of application: 13.06.2018
(73) Proprietor: Luxembourg Institute of Science and Technology (LIST), 4362 Esch-sur-Alzette (LU)
(72) Inventor: PRINTZ, Bruno, 57330 Escherange (FR)
(74) Representative: Lecomte & Partners
(86) International application number: PCT/EP2016/068511
(87) International publication number: WO 2017/025401

(56) References cited:
- WO-A1-2015/013808
- DE-A1- 3 312 214
- US-A1- 2015 005 172
- Renato Iori ET AL: "Immobilization of Myrosinase (Thioglucoside glucohydrolase EC 3.2.3.1)", Biotechnology Letters, 1 August 1988 (1988-08-01), pages 575-578, XP55297809, Retrieved from the Internet: URL:http://rd.springer.com/content/pdf/10. 1007/BF01027132.pdf [retrieved on 2016-08-25]
- ONOFRIO LEONI ET AL: "Immobilization of myrosinase on membrane for determining the glucosinolate content of cruciferous material", JOURNAL OF AGRICULTURAL AND FOOD CHEMISTRY, vol. 39, no. 12, 1 December 1991 (1991-12-01), pages 2322-2326, XP55297803, US ISSN: 0021-8561, DOI: 10.1021/jf00012a046
- ONOFRIO LEONI ET AL: "Hydrolysis of glucosinolates using nylon-immobilized myrosinase to produce pure bioactive molecules", BIOTECHNOLOGY AND BIOENGINEERING, vol. 68, no. 6, 20 June 2000 (2000-06-20), pages 660-664, XP55217496, ISSN: 0006-3592, DOI: 10.1002/(SICI)1097-0290(20000620)68:6<660: :AID-BIT9>3.0.CO;2-L
- ILARIA BRASCHI ET AL: "Activity of myrosinase fromseeds immobilized into Ca-polygalacturonate as a simplified model of soil-root interface mucigel", PLANT AND SOIL ; AN INTERNATIONAL JOURNAL ON PLANT-SOIL RELATIONSHIPS, KLUWER ACADEMIC PUBLISHERS, DO, vol. 339, no. 1 - 2, 15 September 2010 (2010-09-15), pages 209-218, XP019873704, ISSN: 1573-5036, DOI: 10.1007/S11104-010-0569-X
- DARJANA IVETIC ET AL: "Immobilization of [beta]-glucosidase onto mesoporous silica support: Physical adsorption and covalent binding of enzyme", JOURNAL OF THE SERBIAN CHEMICAL SOCIETY, vol. 79, no. 5, 1 January 2014 (2014-01-01), pages 533-543, XP55213525, ISSN: 0352-5139, DOI: 10.2298/JSC131004154I
- GOMEZ J M ET AL: "Immobilization of Î-glucosidase in fixed bed reactor and evaluation of the enzymatic activity", BIOPROCESS AND BIOSYSTEMS ENGINEERING, SPRINGER, BERLIN, DE, vol. 35, no. 8, 3 April 2012 (2012-04-03), pages 1399-1405, XP035106302, ISSN: 1615-7605, DOI: 10.1007/S00449-012-0728-Y
- MARI M ET AL: "In vitro activity of glucosinolate-derived isothiocyanates against postharvest fruit pathogens", ANNALS OF APPLIED BIOLOGY, ASSOCIATION OF APPLIED BIOLOGISTS, WELLESBOURNE, GB, vol. 123, no. 1, 1 January 1993 (1993-01-01), pages 155-164, XP001208100, ISSN: 0003-4746
- SONG-WEI XU ET AL: "Preparation and Catalytic Properties of Novel Alginate-Silica-Dehydrogenase Hybrid Biocomposite Beads", INDUSTRIAL & ENGINEERING CHEMISTRY RESEARCH, vol. 45, no. 2, 1 January 2006 (2006-01-01), pages 511-517, XP55213750, ISSN: 0888-5885, DOI: 10.1021/ie050940y cited in the application
- MIGUEL E ALONSO-AMELOT ET AL: "Kinetics of the Natural Evolution of Hydrogen Cyanide in Plants in Neotropical Pteridium arachnoideum and its Ecological Significance", JOURNAL OF CHEMICAL ECOLOGY, KLUWER ACADEMIC PUBLISHERS-PLENUM PUBLISHERS, NE, vol. 31, no. 2, 1 February 2005 (2005-02-01), pages 315-331, XP019370442, ISSN: 1573-1561, DOI: 10.1007/S10886-005-1343-Z

## Description

### Technical field

The invention is directed to a preparation able to produce a biopesticide and/or repellent for controlling plant pathogens, said preparation comprising an enzyme capable to transform a substrate into a biopesticide and/or repellent product which will subsequently control plant pathogens. The invention also concerns the implementation of such preparation within a pest protection apparatus in order to make use of such preparation in larger scale, for example in greenhouses and/or in private or market gardening.

### Background art

Pest management is currently largely performed with synthetic chemicals, but the development of alternative and eco-friendly methods to control pests are encouraged.

The field of the biocontrol includes various techniques of integrated pest management such as macro-organisms, micro-organisms, pheromones, kairomones and natural substances originating from animals, minerals and plants to control pest and pathogens. In 2014, Sahayaraj K. summarized the current use of the nanotechnologies in the domain of the plant protection. The few data available on this topic brings to the conclusion that only few research all over the world are currently working with this emerging problematic although there is an urgent need of modern approaches of pest management (Sahayaraj K., Adv. Plant Biopest. (chapter 14), 2014, 279-293).

Substances from various plant families have been reported to exert a repelling activity against fungus, bacteria, nematodes or insects and can be successfully used in the treatment/control of pests (Murthy N.B.K., et al., Indian J. Exp. Biol., 1974, 12, 208-209 and Regnault-Roger C., et. al., Biopesticides d'origine végéta/e (2ème edition), 2008**.**) In particular, sulphur containing molecules such as those produced among other by the families *Brassicaceae* and members of the genus *Allium* which are enzymatically produced from non-toxic precursors are toxic at low level for a wide range of organisms (Ahuja I., et al., Agron. Sustain. Dev., 2010, 30, 311-348; Auger J., et al., Ecologie, 1994, 93-101; Cutler H.G., et al., Biologically Active Natural Products: Agrochemicals, 1999).

The mixing of biopesticide precursor with active enzymes in liquid form to produce repellent is known to the art and has been proposed for the production of glucosinolate products such as nitrile, thiocyanate or isothiocyanate, and/or mixture (WO 2015/013808 A1). Also known in the art is the use of a two-part pesticide precursor system comprising a glucosinolate concentrate on one side and an active myrosinase complex on the other side both in dry form. When both components are mixed with water, the glucosinolate breakdown products are released to control pests (US 2015/0005172 A1). However, the lifespan of active enzymes in the environment is relatively low, which considerably hinders the reusability of the enzymes. In addition, the substrates are commonly hydrophilic, making them easily leachable and not accessible to the enzymes.

Enhancing the lifespan of enzymes has been successfully performed in the domains of the biocatalysis, biofuels, enzyme-controlled drug delivery and the biosensors by immobilizing the enzymes into silica mesoporous material (Carlsson N., et al., Adv. Colloid Interface Sci., 2013, 205, 339-360; Popat A., et al., Nanoscale, 2011, 3, 2801-2818; Wang Y., et al., Chem. Mater., 2005, 17, 953-961). Mesoporous material relates to silica-based material with a pore diameter sufficient to allow the penetration of the enzyme. It can be in the form of SBA-15 (Santa Barbara-15), SBA-16, MCM-41 (Mobil Crystalline Materials-41) or all other type of mesoporous silica sieves/spheres/cages (FSM-16 (Folded Sheet Mesoporous-16), MCM-48, FDU-12 (Fudan University-12), MCF (Mesostructured Cellular Foam), SMS (Sponge Mesoporous Silica), mesoporous carbon, PMOs (Periodic Mesoporous Organosilica), Meso-MOFs (meso-Metal Organic Frameworks) (Zhou Z., et al., Top Catal., 2012, 55, 1081-1100). The pore structure may be folded-sheet, 2D hexagonal channels, cubic, spherical cages, mesocellular foam, sponge-like mesoporous silica, 3D cubic cages. The synthesis of the mesoporous silica is preferentially done in acidic conditions and with the triblock copolymer (Pluronic®P123) as template and tetraethyl orthosilicate (TEOS) as silica source. However, the use of other templates such as among other the triblock copolymer (Pluronic®F127), CTMA (cetyltrimethylammonium) (CₙH₂ₙ₊₁(CH₃)N⁺, n=8-18), CTAB (cetyltrimethylammonium bromide) and other silica source including among other (3-aminopropyl)trimethoxysilane (APTMOS), sodium silicate or tetramethyl orthosilicate (TMOS) may also give interesting properties to the silica mesoporous material (Zhou Z., et al., Top Catal., 2012, 55, 1081-1100).

Enzyme immobilization into the silica matrix can be carried out through various techniques including cross-linking methods, covalent binding, physical adsorption, encapsulation and entrapment (CN 101451133 B). Carboxylethyl or aminopropyl functionalization of the mesoporous silica also appears as a good technique for improving the catalytic activities of the silica-based biocomposite (Lei C., et al., J. Am. Chem. Soc., 2002, 124, 11242-11243). Functionalization of the silica-material can further be used for covalent linking of the enzyme on the derivatized-silica.

The synthesis of hydrophilic gels can result from the gelation of various natural compounds such as agarose, agar-agar, alginate, pectin, starch or gelatine taken alone or in mixes. They allow the diffusion of small hydrophilic molecules and prevent the diffusion of large particles such as silica mesoporous materials that retain blocked in the polymeric network. Microencapsulation of silica in alginate had remarkable advantages of sustained-release of compounds and stability under different pH values, different temperatures, and UV irradiation. The presence of the hydrophilic gel can further increase the stability of the material upon ageing and limit enzyme leaching (Coradin T., et al., Comptes Rendus Chim., 2003, 6, 147-152). Biocomposite incorporation in hydrophilic gels such as alginate showed much better performance due to the more homogeneous distribution of silica particles in the composite material (Xu S. et al., Ind. Eng. Chem. Res., 2006, 45, 511-517).

Renato I. et al. (Biotechnology Letters 01.08.1988) disclose the enzyme myrosinase which was extracted from white mustard seed (Sinapis alba).

Onofrio L. et al. (Journal of Agricultural and Food Chemistry, vol. 39, n°12, 01.12.1991) disclose the immobilisation of myrosinase on various membranes, such as pig intestine, teflon and nylon 6.6.

Onofrio L. et al. (Biotechnology and Bioengineering, vol. 68, n°6, 020.06.2000) disclose the hydrolysis of glucosinolates using nylon 6.6-immobilised myrosinase in order to produce bioactive molecules, such as isothiocyanates.

Braschi I. et al. (An International Journal on Plant-Soil relationships, Kluwer Academic Publishers, DO, vol. 339, n°1-2, 15.09.2015) disclose the immobilisation of myrosinase in calcium polygalacturonate to study the behaviour of the enzyme at the soil root interface, in particular with regard to the hydrolysis of sinigrin to allylisothiocyanate.

Ivetic D. et al. (Journal of the Serbian Chemical Society, vol. 79, n° 5, 01.01.2014) disclose the immobilisation of β-glucosidase on mesoporous silica by cross-linking with glutaraldehyde.

Gomez JM. et al. (Bioprocess and Biosystems Engineering, Springer, Berlin, DE, vol. 35, n° 8, 03.04.2012) disclose the immobilisation of β-glucosidase on mesoporous silica. Enzyme activity was maintained in a fixed bed reactor for a prolonged period of time.

Mari M. et al. (Annals of Applied Biology, Association of Applied Biologists, Wellesbourne,GB, vol. 123, n°1, 01.01.1993) disclose the production of isothiocyanates from glucosinolates by means of myrosinase-catalysed hydrolysis and the activity of such isothiocyanates against fungal post-harvest fruit pathogens.

Xu S-W et al. (Industrial & Engineering Chemistry Research, vol. 45, n°2, 01.01.2006) disclose a method of providing enzymes in hydrophilic gels in order to show better performance.

Alonso-Amelot M.E. et al. (Journal of Chemical Ecology, Kluwer Academic Publishers-Plenum Publishers, NE, vol. 31, n° 2, 01.02.2005) disclose the release of hydrogen cyanide from cyanogenic plants. Such plants release hydrogen cyanide to protect themselves from attack by chewing herbivores.

In the present invention, these parts (mixing of a precursor with active enzymes in liquid form to produce biopesticide and/or repellent product, enhancing the lifespan of said enzymes, use of hydrophilic gels) are merged together in order to produce for the first time active mesoporous materials encapsulated in a hydrophilic gel in order to produce *in situ* a constant flux of biopesticide and/or repellent product.

On the other hand, patent application published US 2012/0079625 A1 relates to a method for protecting living plants from harmful insects via a sheet-like structure impregnated with an insecticide which is from synthetic origin.

### Summary of invention

### Technical Problem

The invention has for technical problem to provide a fibre with a preparation, able to produce a biopesticide and/or repellent for controlling plant pathogens, which is non-toxic and easy to handle. This will confer interesting storage and manipulation properties while keeping the biopesticide and/or repellent properties of such fibre.

### Technical solution

The first object of the present invention is a fibre according to claim 1. Preferred embodiments are recited in the following paragraphs and in dependent claims.

In one embodiment, the at least one nanoporous material is a mesoporous material.

In one embodiment, said hydrophilic gel is made of agarose, agar-agar, alginate, pectin, starch and/or gelatine, preferentially made of alginate.

In one embodiment, said at least one active enzyme from the group of glycosidase is thioglucosidase.

In one embodiment, said at least one active enzyme immobilized within said at least one nanoporous material is covalently bounded to said at least one nanoporous material, physically adsorbed to said at least one nanoporous material, encapsulated within said at least one nanoporous material or entrapped within said at least one nanoporous material.

In one embodiment, said active enzyme immobilized within said at least one nanoporous material is cross-linked together with glutaraldehyde or with 1-ethyl-3-(3-dimethylaminopropyl)carbodiimide and *N-*hydroxysuccinimide.

In one embodiment, said at least one nanoporous material is a silica-based mesoporous material.

In one embodiment, said silica-based mesoporous material comprises tetraethyl orthosilicate and/or tetramethyl orthosilicate and/or methyltrimethoxysilane and/or (3-aminopropyl)trimethoxysilane.

In one embodiment, the preparation has a pH comprised between 4 and 11.

In one embodiment, the preparation further comprises at least one mineral and/or at least one cofactor.

In one embodiment, said preparation is adapted to receive said at least one precursor, at least one mineral and/or at least one cofactor.

In one embodiment, said at least one mineral and/or said at least one cofactor is phosphate buffer or ascorbic acid.

In one embodiment, said phosphate buffer is at a concentration comprised between 10 mM and 500 mM, preferentially at a concentration comprised between 50 mM and 250 mM, more preferentially at a concentration comprised between 75 mM and 150 mM, even more preferentially at a concentration of 100 mM.

In one embodiment, said ascorbic acid is at a concentration comprised between 50 µM and 1500 µM, preferentially at a concentration comprised between 150 µM and 1000 µM, more preferentially at a concentration comprised between 250 µM and 750 µM, even more preferentially at a concentration of 500 µM.

In one embodiment, the preparation is dehydrated and/or rehydrated.

The at least one preparation is further coated with a hydrophobic layer.

In one embodiment, said hydrophobic layer is made of 3-aminopropyl)triethoxysilane, (3-mercaptopropyl)triethoxysilane, succinic anhydride, alkylketene dimer, 3-isopropenyl-α-α-dimethylbenzyl isocyanate, *m*-phenylene bismaleimide, vinyl trialkoxysilane, 3-metacryloyloxy propyl trimetoxysilane or any other.

In one embodiment, said fibre is a natural fibre, preferentially hemp, flax, nettle, cotton, jute, ramie, sisal or any other, more preferentially hemp or flax.

It is a second object of the present invention to disclose a pest protective apparatus comprising at least one fibrous network and/or at least one fibrous cover according to claim 10. Said pest protective apparatus is remarkable in that said at least one fibrous network and/or said at least one fibrous cover is made of fibres adapted to control plant pathogens, in accordance with the first object of the present invention.

In one embodiment, the cartridge further comprises at least one mineral and/or at least one cofactor.

In one embodiment, the cartridge is adapted to receive said at least one precursor, at least one mineral and/or at least one cofactor.

In one embodiment, said at least one mineral and/or said at least one cofactor is phosphate buffer or ascorbic acid.

In one embodiment, said phosphate buffer is at a concentration comprised between 10 mM and 500 mM, preferentially at a concentration comprised between 50 mM and 250 mM, more preferentially at a concentration comprised between 75 mM and 150 mM, even more preferentially at a concentration of 100 mM.

In one embodiment, said ascorbic acid is at a concentration comprised between 50 µM and 1500 µM, preferentially at a concentration comprised between 150 µM and 1000 µM, more preferentially at a concentration comprised between 250 µM and 750 µM, even more preferentially at a concentration of 500 µM.

It is a third object of the present invention to disclose the teachings of a method for controlling plant pathogens with a preparation able to produce a biopesticide and/or repellent for controlling plant pathogens according to claim 11. The first step of activating at least one precursor may also be, for example, the catalytic splitting of said at least one precursor.

In one embodiment, said at least one precursor is comprised in the preparation able to produce a biopesticide and/or repellent for controlling plant pathogens, the preparation preferentially also comprising at least one mineral and/or at least one cofactor.

In one embodiment, said at least one precursor is added to the preparation able to produce a biopesticide and/or repellent for controlling plant pathogens the preparation preferentially comprising at least one mineral and/or at least one cofactor.

In one embodiment, said at least one precursor is added with also at least one mineral and/or at least one cofactor to the preparation able to produce a biopesticide and/or repellent for controlling plant pathogens in accordance with the first object of the present invention.

The preparation able to produce a biopesticide and/or repellent for controlling plant pathogens is on a fibre adapted to control plant pathogen in accordance with the first object of the present invention, said fibre being preferentially comprised into a pest protective apparatus in accordance with the second object of the present invention.

### Advantages of the invention

The invention is particularly interesting in that the precursor or the precursors of the biopesticide and/or repellent product which is provided by the preparation is chosen among the inactive and non-toxic forms of the potential organic derivatives. The immobilization of the enzyme into a nanoporous material, preferably a silica nanoporous material, more preferably a silica-based mesoporous material, will further enhance the stability of the core of the preparation able to produce a biopesticide and/or repellent for controlling plant pathogens and will further enhance the productivity of the compound with biopesticide and/or repellent properties.

### Brief description of the drawings

Figure 1: Preparation able to produce a biopesticide and/or repellent for controlling plant pathogens.
Figure 2: Fibres adapted to control plant pathogens.
Figure 3: Pest protective apparatus.

### Description of an embodiment

The present invention relates to a pest management biocontrol system made of active biocatalysts able to produce natural repellents and/or biopesticides by means of controlled enzymatic reactions.

The present invention offers to the user an easy-to-handle and safe way to control plant pest by producing a constant flux of biopesticide and/or repellent products, being insecticides, fungicides, bactericides and/or nematicides.

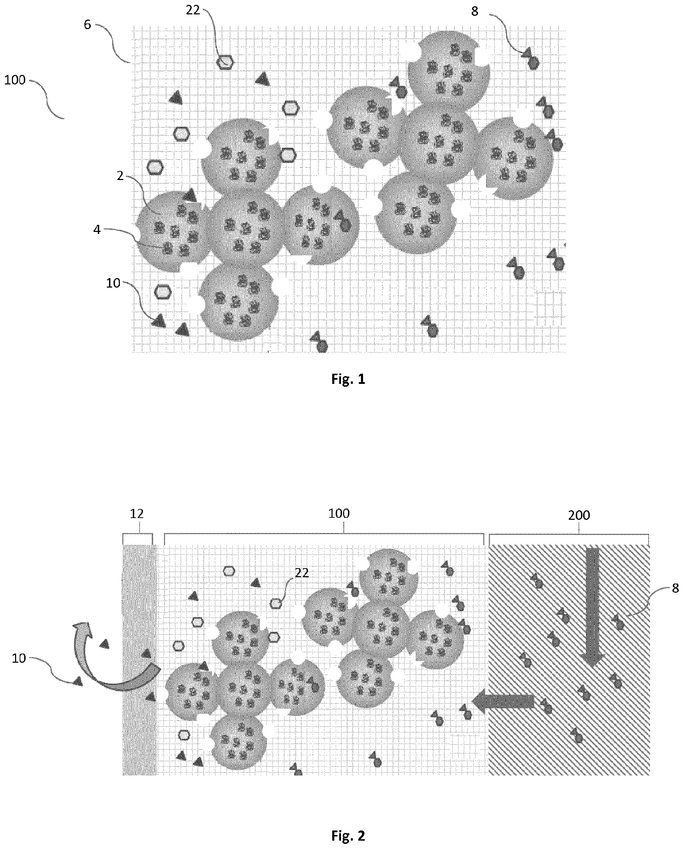

Figure 1 depicts a preparation 100, able to produce a biopesticide and/or repellent for controlling plant pathogens, according to the present invention.

The biopesticide and/or repellent compounds produced are those involved in the natural reaction of the plants when facing a pathogen attack. They have a low half-life in the environment and do not affect the quality of the plants which are treated.

The present invention relates to an innovative product that entraps enzymes 4 into a nanoporous material 2, in particular a silica sphere, preferably into a mesoporous silica matrix, more particularly into a silica-based mesoporous material, the whole being glued or embedded into a hydrophilic gel 6, to degrade natural molecules or extracts into products 10 having biopesticide and/or repellent activity.

The mesoporous materials 2 of the present invention are ordered silica-based mesoporous particles with a narrow pore size distribution, a well-defined pore geometry and a well-defined pore connectivity.

The pore size distribution and the global geometry of the mesoporous materials 2 are defined according to the properties of the biopesticide-producing enzymes and/or repellent-producing enzymes that are immobilized into the mesoporous structure.

The pore size of the silica-based mesoporous material is comprised between 5 nm and 30 nm, more particularly 5 and 15 nm for thioglucosidase.

The immobilization procedure should allow to optimize the catalytic activities of the enzymes in comparison with the free enzymes and to enhance their reusability in order to produce a constant and sufficient flux of biopesticide and/or repellent product allowing a sufficient biopesticide and/or repellent activity against the targeted pathogens.

The silica-based mesoporous material will be synthesized in acidic conditions using a silica precursor that could be among other tetraethyl orthosilicate (TEOS) and/or tetramethyl orthosilicate (TMOS) and/or methyltrimethoxysilane (MTMOS) and/or (3-aminopropyl)triemthoxysilane (APTMOS), in combination with tri-block copolymer mixtures such as Pluronic®F127 and/or Pluronic®F123 as structure-directing agents. Numerous methods are known by the skilled person in the art to achieve this synthesis.

The mesoporous structure can be produced hydrothermally and/or by solgel synthesis and can be functionalized.

The enzymes 4 that may be immobilized into the mesoporous material are those involved in the natural response of the plants when facing a pathogen attack. The biopesticide and/or repellent compounds produced during the enzymatic reaction can be a bactericide, a fungicide, an insecticide and/or a nematicide.

The enzyme from the glycosidase, the oxidoreductases, the transferases, the hydrolases, the lyases, the isomerases and the ligases can be immobilized into the mesoporous material.

Preferred transferases are those belonging to the glycosidases class, i.e. the enzymes hydrolysing O-glycosyl and S-glycosyl compounds.

Preferred lyases are, for example, carbon-sulfur lyases, in particular alliin lyase (also known as alliinase).

Enzymes from the PF10604 family, i.e. the lachrymatory-factor synthase, can also be used in the present invention.

A preferred glycosidase is thioglucosidase. This is the enzyme of choice which has been tested for transforming a precursor into a biopesticide and/or repellent product.

The immobilization procedure can be performed through the following techniques known in the art: cross-linking methods, covalent binding, physical adsorption, encapsulation and entrapment.

The immobilization includes the penetration of the enzymes inside the mesoporous material, through the nanopores and an eventual step of cross-linkage between the enzymes using gluturaldehyde or 1-ethyl-3-(3-dimethylaminopropyl)carbodiimide and *N*-hydroxysuccinimide. Numerous methods are known by the skilled person in the art to achieve the immobilization step.

The mesoporous biocatalyst will then be embedded in a hydrophilic gel 6 made of agarose, agar-agar, alginate, pectin, starch, gelatine taken alone or in mixes, preferentially alginate.

The use of alginate allows a gelation without heating. Only CaCl₂ must be added for the gelation step.

The hydrophilic gel 6 allows the diffusion of the products of the enzymatic reaction, the diffusion of the substrates 8 of the enzymes 4 and the confinement of all chemicals required to maintain the enzymes 4 in a working status.

The hydrophilic gel 6 may contain all minerals and cofactors indispensable for the enzymatic activities. This can be a phosphate butter (pH = 6.1) at a concentration comprised between 10 mM and 500 mM, preferentially at a concentration of 100 mM and/or ascorbic acid at a concentration comprised between 50 µM and 1500 µM, preferentially at a concentration of 500 µM.

In case the minerals and cofactors are not added before the gelation step, they can be added by diffusion within the gel by soaking into an aqueous buffer comprising these compounds.

The pH of the preparation 100 able to produce biopesticide and/or repellent for controlling plant pathogens is preferably comprised between 4 and 11. In particular, the pH of the hydrophilic gel allows an optimal activity of the immobilized enzymes.

The hydrophilic gel with the silica-based mesoporous material embedded within said hydrophilic gel, said silica-based mesoporous comprising immobilized thioglucosidase, forms a biopesticide and/or repellent preparation 100 adapted to control plant pathogen.

Upon addition of the substrate 8 for the enzyme 4 and upon addition of water, and, upon addition of minerals and cofactors for activating the enzyme 4 if they are not formerly included within said hydrophilic gel 6, the enzyme will proceed to the synthesis of a product 10 which will present biopesticide and/or repellent properties. This product 10 will be released and will act to eliminate (in case of biopesticide activity) and/or to keep away (in case of repellent activity) the pest and/or the plant pathogens from the living plants, such as bacteria, fungi, insects, bugs, and/or nematodes.

The scheme on figure 1 represents in particular a precursor 8 composed of two chemical moieties, one being the biopesticide and/or the repellent product 10, and the other being a compound 22.

Preferentially, the product 10 of the enzymatic reaction is a volatile organic compound.

The biopesticide and/or repellent preparation 100 can further be coated onto fibres 200 or natural fibres. This is schematically depicted on figure 2.

Such fibres might be for example hemp, flax, nettle, cotton, jute, ramie, sisal, and/or any other.

Preferentially, the fibres are hemp or flax.

The substrate 8 of the enzyme 4 is able to diffuse within the fibre 200. This will allow said substrate 8 to reach the preparation 100 able to produce biopesticide and/or repellent for controlling plant pathogens in order to be processed by the enzyme 4.

In order to protect such fibres 200 from drying, a protective layer, preferentially a hydrophobic layer 12 will be coated over the fibres 200 containing a coating of the preparation 100 able to produce biopesticide and/or repellent for controlling plant pathogens.

Such hydrophobic layer 12 is also configured to be permeable to the biopesticide and/or repellent product 10.

The hydrophobic layer 12 may be (3-aminopropyl)triethoxysilane, (3-mercaptopropyl)triethoxysilane, succinic anhydride, alkylketene dimer, 3-isopropenyl-α-α-dimethylbenzyl isocyanate, m-phenylene bismaleimide, vinyl trialkoxysilane, 3-metacryloyloxy propyl trimetoxysilane and/or any other.

The moiety 22, resulting from the cleavage of the precursor 8, stays therefore or diffuses slowly within the preparation 100 able to produce biopesticide and/or repellent for controlling plant pathogens and does not therefore pollute the environment.

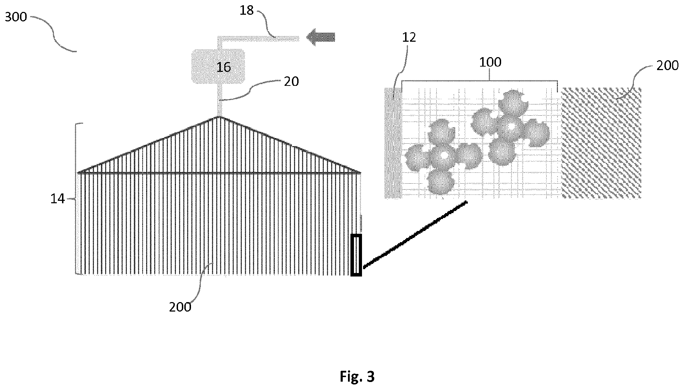

As indicated on figure 3, the fibres 200 with the biopesticide and/or repellent preparation 100 might also be incorporated within a pest protective apparatus 300, more particularly by coating onto at least one fibrous network and/or at least one fibrous cover 14 of said pest protective apparatus 300.

The pest protective apparatus 300 of the present invention may also comprise means for distributing water to the at least one fibrous network and/or the at least one fibrous cover 14, said means preferably comprising a cartridge 16. Said cartridge 16 is configured for being in fluid connection with said at least one fibrous network and/or said at least one fibrous cover 14 and for being fed with water. Said cartridge 16 may comprise an inlet 18 and an outlet 20.

Depending whether the pest protective apparatus 300 comprises or does not comprise the cartridge 16, said apparatus is configured either to transform at least one precursor 8, which is incorporated to the preparation 100 able to produce biopesticide and/or repellent for controlling plant pathogens by diffusion through the fibre 200, into a biopesticide and/or a repellent product 10; or to incorporate the precursor 8 into said cartridge 16.

Depending whether the pest protective apparatus 300 comprises or does not comprise the cartridge 16, the at least one mineral and/or the at least one cofactor indispensable for the enzyme activity are incorporated either directly within the hydrophilic gel 6 of the biopesticide and/or repellent preparation 100; or within the cartridge 16.

In the case of the preparation 100 able to produce biopesticide and/or repellent for controlling plant pathogens, in the case of the fibres, as well as in the case of the pest protective apparatus of the present invention, the enzymatic reaction is triggered by the addition of water, which plays the role of the solvent of the reaction, bringing subsequently the substrate 8 into contact with the fibres 200 of the preparation 100 able to produce biopesticide and/or repellent for controlling plant pathogens, and more particularly, into contact with the active site of the enzyme 4.

The second utility of water is to bring to the enzyme 4 the at least one mineral and/or the at least one cofactor indispensable for its activity.

Water can come from the rain and/or from artificial means, such for example a drain pump, a hosepipe and/or an irrigation system, and can be channelled to contact the preparation 100 able to produce biopesticide and/or repellent for controlling plant pathogens, triggering subsequently the enzymatic reaction.

When the pest protective apparatus 300 comprises a cartridge 16, the flux of water reaches the cartridge 16 through the inlet 18 which is provided on the cartridge.

Once the enzymatic reaction is over, the product 10, which presents biopesticide and/or repellent properties, is released. Generally, the product is a volatile organic compound.

This product acts to eliminate (in case of biopesticide activity) and/or to keep away (in case of repellent activity) the pests and/or the plant pathogens from the living plants, such as bacteria, fungi, insects, bugs, and/or nematodes.

The biopesticide and/or repellent product 10 resulting from the activation of the precursor by addition of water to the preparation 100 taught in the present invention is applied to plants, preferentially vegetables and/or fruits, in order to prevent pest attacks.

## Claims

1. Fibre (200) adapted to control plant pathogens, **characterized in that** said fibre (200) is coated with at least one preparation (100) able to produce a biopesticide and/or repellent for controlling plant pathogens, said preparation (100) comprising at least one nanoporous material (2), at least one biopesticide and/or repellent precursor (8) comprising O-glycosyl and S-glycosyl type compounds, and at least one active enzyme (4) selected from the group of glycosidase, said at least one active enzyme (4), hydrolyzing O-glycosyl and S-glycosyl compounds, being configured to transform the at least one biopesticide and/or repellent precursor (8) into a biopesticide and/or repellent product (10), wherein said at least one active enzyme (4) is immobilized within said at least one nanoporous material (2), wherein said at least one nanoporous material (2) is embedded within one hydrophilic gel (6), and wherein said preparation is further coated with an hydrophobic layer.

2. Fibre (200) according to claim 1, **characterized in that** said fibre is hemp or flax.

3. Fibre (200) according to any one of claims 1-2, **characterized in that** said hydrophilic gel (6) is made of agarose, agar-agar, alginate, pectin, starch and/or gelatine, preferentially made of alginate.

4. Fibre (200) according to any one of claims 1-3, **characterized in that** said at least one active enzyme (4) is thioglucosidase.

5. Fibre (200) according to any one of claims 1-4, **characterized in that** said at least one active enzyme (4) is covalently bond to, physically adsorbed to, encapsulated within, or entrapped within said at least one nanoporous material (2).

6. Fibre (200) according to any one of claims 1-5, **characterized in that** said at least one active enzyme (4) is cross-linked with glutaraldehyde or with 1-ethyl-3-(3-dimethylaminopropyl)carbodiimide and N-hydroxysuccinimide.

7. Fibre (200) according to any one of claims 1-6, **characterized in that** said at least one nanoporous material (2) is a silica-based mesoporous material.

8. Fibre (200) according to any one of claims 1-7, **characterized in that** said preparation (100) further comprises at least one mineral and/or at least one cofactor.

9. Fibre (200) according to claim 8, **characterized in that** said at least one mineral and/or said at least one cofactor is phosphate buffer or ascorbic acid.

10. Pest protective apparatus (300), comprising at least one fibrous network and/or at least one fibrous cover (14),
**characterized in that** said at least one fibrous network and/or said at least one fibrous cover (14) is made of fibres (200) adapted to control plant pathogens, said fibres (200) being coated with at least one preparation (100) able to produce a biopesticide and/or repellent for controlling plant pathogens, said preparation (100) comprising at least one nanoporous material (2) and at least one active enzyme (4), said enzyme is as defined according to any one of claims 1, 4 to 6, **characterized in that** at least one biopesticide and/or repellent precursor (8) comprising O-glycosyl and S-glycosyl type compounds is either comprised in the preparation (100) or is incorporated into a cartridge (16), said cartridge (16) being included in means for distributing water to the fibrous network and/or fibrous cover (14) and configured for being in fluid connection with said at least one fibrous network and/or said at least one fibrous cover (14) and for being fed with water.

11. A method for controlling plant pathogens with a preparation (100) comprising at least one nanoporous material (2) and at least one active enzyme (4) as defined according to any one of claims 1, 4 to 6, said at least one active enzyme (4) being configured to transform at least one biopesticide and/or repellent precursor (8) comprising O-glycosyl and S-glycosyl type compounds into a biopesticide and/or repellent product (10), **characterized in that** said method comprises the following steps of:
a) activating the at least one biopesticide and/or repellent precursor (8) by addition of water to the preparation (100) coated onto a fibre (200) adapted to control plant pathogens, the at least one biopesticide and/or repellent precursor (8) is either comprised in the preparation (100) or is incorporated into a cartridge (16) of the pest protective apparatus (300) in accordance with claim 10;
b) bringing subsequently the precursor (8) into contact with the fibres (200) coated with the preparation (100), preferentially with active site of the enzyme (4), for producing a biopesticide and/or repellent product (10) for controlling plant pathogens, and
c) applying the biopesticide and/or repellent product (10) resulting from step (b) to plants, preferentially vegetables and/or fruits, in order to prevent pest attacks.

12. The method for controlling plant pathogens according to claim 11, **characterized in that** said at least one biopesticide and/or repellent precursor (8) is either comprised in the preparation (100) or is incorporated into the cartridge (16) with at least one mineral and/or at least one cofactor.

## Patentansprüche

1. Faser (200), die dafür ausgelegt ist, Pflanzenpathogene einzudämmen, **dadurch gekennzeichnet, dass** die Faser (200) mit mindestens einem Präparat (100) beschichtet ist, das in der Lage ist, ein Biopestizid und/oder Repellent zum Eindämmen von Pflanzenpathogenen zu produzieren, wobei das Präparat (100) umfasst: mindestens ein nanoporöses Material (2), mindestens einen Biopestizid- und/oder Repellentvorläufer (8), der Verbindungen des Typs O-Glycosyl und S-Glycosyl umfasst, und mindestens ein aktives Enzym (4), ausgewählt aus der Glycosidasegruppe, wobei das mindestens eine aktive Enzym (4), das O-Glycosyl- und S-Glycosylverbindungen hydrolysiert, dafür ausgelegt ist, den mindestens einen Biopestizid- und/oder Repellentvorläufer (8) in ein Biopestizid- und/oder Repellentprodukt (10) umzuwandeln, wobei das mindestens eine aktive Enzym (4) innerhalb des mindestens einen nanoporösen Materials (2) immobilisiert ist, wobei das mindestens eine nanoporöse Material (2) in ein hydrophiles Gel (6) eingebettet ist, und wobei das Präparat ferner mit einer hydrophoben Schicht beschichtet ist.

2. Faser (200) nach Anspruch 1, **dadurch gekennzeichnet, dass** die Faser Hanf oder Flachs ist.

3. Faser (200) nach einem der Ansprüche 1-2, **dadurch gekennzeichnet, dass** das hydrophile Gel (6) aus Agarose, Agar-Agar, Alginat, Pektin, Stärke und/oder Gelatine, vorzugsweise aus Alginat hergestellt ist.

4. Faser (200) nach einem der Ansprüche 1-3, **dadurch gekennzeichnet, dass** das mindestens eine aktive Enzym (4) Thioglucosidase ist.

5. Faser (200) nach einem der Ansprüche 1-4, **dadurch gekennzeichnet, dass** das mindestens eine aktive Enzym (4) an das mindestens eine nanoporöse Material (2) kovalent gebunden, physikalisch adsorbiert, darin einkapselt oder darin eingefangen ist.

6. Faser (200) nach einem der Ansprüche 1-5, **dadurch gekennzeichnet, dass** das mindestens eine aktive Enzym (4) mit Glutaraldehyd oder mit 1-Ethyl-3-(3-dimethylaminopropyl)carbodiimid und N-Hydroxysuccinimid vernetzt ist.

7. Faser (200) nach einem der Ansprüche 1-6, **dadurch gekennzeichnet, dass** das mindestens eine nanoporöse Material (2) ein mesoporöses Material auf Siliciumdioxidgasis ist.

8. Faser (200) nach einem der Ansprüche 1-7, **dadurch gekennzeichnet, dass** das Präparat (100) ferner mindestens ein Mineral und/oder mindestens einen Cofaktor umfasst.

9. Faser (200) nach Anspruch 8, **dadurch gekennzeichnet, dass** das mindestens eine Mineral und/oder der mindestens eine Cofaktor Phosphatpuffer oder Ascorbinsäure ist.

10. Vor Schädlingen schützende Vorrichtung (300), mindestens ein faseriges Netz und/oder mindestens eine faserige Abdeckung (14) umfassend,
**dadurch gekennzeichnet, dass** das mindestens eine faserige Netz und/oder die mindestens eine faserige Abdeckung (14) aus Fasern (200) hergestellt ist, die dafür ausgelegt sind, Pflanzenpathogene einzudämmen, wobei die Fasern (200) mit mindestens einem Präparat (100) beschichtet sind, das in der Lage ist, ein Biopestizid und/oder Repellent zum Eindämmen von Pflanzenpathogenen zu produzieren, wobei das Präparat (100) umfasst: mindestens ein nanoporöses Material (2) und mindestens ein aktives Enzym (4), wobei das Enzym ist wie gemäß einem der Ansprüche 1, 4 bis 6 definiert, **dadurch gekennzeichnet, dass** der mindestens eine Biopestizid- und/oder Repellentvorläufer (8), der Verbindungen des Typs O-Glycosyl und S-Glycosyl umfasst, entweder in dem Präparat (100) enthalten ist oder in einer Kartusche (16) aufgenommen ist, wobei die Kartusche (16) in einer Einrichtung zur Verteilung von Wasser an das faserige Netz und/oder die faserige Abdeckung (14) enthalten ist und dafür ausgelegt ist, mit dem mindestens einen faserigen Netz und/oder der mindestens einen faserigen Abdeckung (14) in Fluidverbindung zu stehen und mit Wasser beschickt zu werden.

11. Verfahren zum Eindämmen von Pflanzenpathogenen mit einem Präparat (100), das mindestens ein nanoporöses Material (2) und mindestens ein aktives Enzym (4) nach einem der Ansprüche 1, 4 bis 6 umfasst, wobei das mindestens eine aktive Enzym (4) dafür ausgelegt ist, mindestens einen Biopestizid- und/oder Repellentvorläufer (8), der Verbindungen des Typs O-Glycosyl und S-Glycosyl umfasst, in ein Biopestizid- und/oder Repellentprodukt (10) umzuwandeln, **dadurch gekennzeichnet, dass** das Verfahren die folgenden Schritte umfasst:
a) Aktivieren des mindestens einen Biopestizid- und/oder Repellentvorläufers (8) durch die Zugabe von Wasser zu dem Präparat (100), mit dem eine Faser (200) beschichtet ist, die dafür ausgelegt ist, Pflanzenpathogene einzudämmen, wobei der mindestens eine Biopestizid- und/oder Repellentvorläufer (8) entweder in dem Präparat (100) enthalten ist oder in einer Kartusche (16) der vor Schädlingen schützenden Vorrichtung (300) nach Anspruch 10 aufgenommen ist;
b) anschließend In-Kontakt-Bringen des Vorläufers (8) mit den mit dem Präparat (100) beschichteten Fasern (200), vorzugsweise mit einer aktiven Stelle des Enzyms (4), um ein Biopestizid- und/oder Repellentprodukt (10) zum Eindämmen von Pflanzenpathogenen zu produzieren, und
c) Anwenden des Biopestizid- und/oder Repellentprodukts (10), das sich aus Schritt (b) ergibt, auf Pflanzen, vorzugsweise auf Gemüse und/oder Obst, um Schädlingsbefall zu verhindern.

12. Verfahren zum Eindämmen von Pflanzenpathogenen nach Anspruch 11, **dadurch gekennzeichnet, dass** mindestens ein Biopestizid- und/oder Repellentvorläufer (8) entweder in dem Präparat (100) enthalten ist oder mit mindestens einem Mineral und/oder mindestens einem Cofaktor in die Kartusche (16) aufgenommen ist.

## Revendications

1. Fibre (200) conçue pour la lutte contre des agents phytopathogènes, **caractérisée en ce que** ladite fibre (200) est enduite d'au moins une préparation (100) qui est capable de produire un biopesticide et/ou un répulsif destiné à la lutte contre des agents phytopathogènes, ladite préparation (100) comprenant au moins une matière nanoporeuse (2), au moins un précurseur de biopesticide et/ou de répulsif (8) qui comprend des composés de type O-glycosylé et S-glycosylé, et au moins une enzyme active (4) qui est choisie parmi le groupe des glycosidases, ladite au moins une enzyme active (4), soumettant à une hydrolyse des composés de type O-glycosylé et S-glycosylé, étant configurée pour transformer ledit au moins un précurseur de biopesticide et/ou de répulsif (8) en un produit (10) sous la forme d'un biopesticide et/ou sous la forme d'un répulsif; dans laquelle ladite au moins une enzyme active (4) est immobilisée au sein de ladite au moins une matière nanoporeuse (2) ; dans laquelle ladite au moins une matière nanoporeuse (2) est enfermée dans un gel hydrophile (6) ; et dans laquelle ladite préparation est en outre enduite d'une couche hydrophobe.

2. Fibre (200) selon la revendication 1, **caractérisée en ce que** ladite fibre représente du chanvre ou du lin.

3. Fibre (200) selon l'une quelconque des revendications 1 à 2, **caractérisée en ce que** ledit gel hydrophile (6) est constitué d'agarose, d'agar-agar, d'alginate, de pectine, d'amidon et/ou de gélatine, de manière préférentielle est constitué d'alginate.

4. Fibre (200) selon l'une quelconque des revendications 1 à 3, **caractérisée en ce que** ladite au moins une enzyme active (4) représente la thioglucosidase.

5. Fibre (200) selon l'une quelconque des revendications 1 à 4, **caractérisée en ce que** ladite au moins une enzyme active (4) est liée de manière covalente à ladite au moins une matière nanoporeuse (2), est adsorbée à cette dernière par voie physique, est encapsulée dans ladite matière ou est enfermée dans ladite matière.

6. Fibre (200) selon l'une quelconque des revendications 1 à 5, **caractérisée en ce que** ladite au moins une enzyme active (4) est soumise à une réticulation avec du glutaraldéhyde ou avec du 1-éthyl-3-(3-diméthylaminopropyl)carbodiimide et du N-hydroxysuccinimide.

7. Fibre (200) selon l'une quelconque des revendications 1 à 6, **caractérisée en ce que** ladite au moins une matière nanoporeuse (2) est une matière mésoporeuse à base de silice.

8. Fibre (200) selon l'une quelconque des revendications 1 à 7, **caractérisée en ce que** ladite préparation (100) comprend en outre au moins une matière minérale et/ou au moins un cofacteur.

9. Fibre (200) selon la revendication 8, **caractérisée en ce que** ladite au moins une matière minérale et/ou ledit au moins un cofacteur représente un tampon de phosphate ou l'acide ascorbique.

10. Appareil de protection (300) contre des organismes nuisibles qui comprend au moins un réseau fibreux et/ou au moins un revêtement fibreux (14), **caractérisé en ce que** ledit au moins un réseau fibreux et/ou ledit au moins un revêtement fibreux (14) est/sont constitués de fibres (200) qui sont conçues pour lutter contre des agents phytopathogènes, lesdites fibres (200) étant enduites avec au moins une préparation (100) qui est capable de produire un biopesticide et/ou un répulsif destiné à la lutte contre des agents phytopathogènes, ladite préparation (100) comprenant au moins une matière nanoporeuse (2) et au moins une enzyme active (4), ladite enzyme étant telle que définie conformément à l'une quelconque des revendications 1, 4 à 6, **caractérisé en ce qu'**au moins un précurseur de biopesticide et/ou de répulsif (8) qui comprend des composés de type O-glycosylé et S-glycosylé, soit est compris dans la préparation (100), soit est incorporé dans une cartouche (16), ladite cartouche (16) étant incluse dans des moyens destinés à distribuer de l'eau sur le réseau fibreux et/ou sur le revêtement fibreux (14) et étant configurée pour être mise en communication fluidique avec ledit au moins un réseau fibreux et/ou ledit au moins un revêtement fibreux (14) et pour être alimentée avec de l'eau.

11. Procédé destiné à la lutte contre des agents phytopathogènes avec une préparation (100) qui comprend au moins une matière nanoporeuse (2) et au moins une enzyme active (4), ladite enzyme étant telle que définie selon l'une quelconque des revendications 1, 4 à 6, ladite au moins une enzyme active (4) étant configurée pour transformer au moins un précurseur de biopesticide et/ou de répulsif (8) comprenant des composés de type O-glycosylé et S-glycosylé en un produit (10) sous la forme d'un biopesticide et/ou sous la forme d'un répulsif, **caractérisé en ce que** ledit procédé comprend les étapes suivantes dans lesquelles :
a) on active ledit au moins un précurseur de biopesticide et/ou de répulsif (8) par addition d'eau à la préparation (100) enduite sur une fibre (200) qui est conçue pour la lutte contre des agents phytopathogènes, ledit au moins un précurseur de biopesticide et/ou de répulsif (8), soit étant compris dans la préparation (100), soit étant incorporé dans une cartouche (16) de l'appareil de protection contre des organismes nuisibles (300) selon la revendication 10 ;
b) on amène par la suite le précurseur (8) en contact avec les fibres (200) enduites avec la préparation (100), de manière préférentielle en contact avec un site actif de l'enzyme (4), afin d'obtenir un produit (10) sous la forme d'un biopesticide et/ou sous la forme d'un répulsif destiné à la lutte contre des agents phytopathogènes ; et
c) on applique sur des plantes, de manière préférentielle sur des légumes et/ou sur des fruits, le produit (10) sous la forme d'un biopesticide et/ou sous la forme d'un répulsif que l'on obtient partir de l'étape (b), dans le but d'empêcher des attaques d'agents nuisibles.

12. Procédé destiné à la lutte contre des agents phytopathogènes selon la revendication 11, **caractérisé en ce que** ledit au moins un précurseur de biopesticide et/ou de répulsif (8), soit est compris dans la préparation (100), soit est incorporé dans une cartouche (16), avec au moins une matière minérale et/ou au moins un cofacteur.
